Europäisches Patentamt

European Patent Office

Office européen des brevets

(11) Publication number: **0 059 521**
**B1**

(12) **EUROPEAN PATENT SPECIFICATION**

(45) Date of publication of patent specification: **02.01.85**

(51) Int. Cl.⁴ **A 61 K 39/39**, A 61 K 9/00

(21) Application number: **82200270.5**

(22) Date of filing: **02.03.82**

(54) **Water-in-oil emulsion for use in the potentation of the immune system of animals.**

(30) Priority: **03.03.81 NL 8101023**

(43) Date of publication of application:
**08.09.82 Bulletin 82/36**

(45) Publication of the grant of the patent:
**02.01.85 Bulletin 85/01**

(84) Designated Contracting States:
**BE DE FR GB IT NL SE**

(56) References cited:
**BE-A- 648 053**
**GB-A-1 171 125**
**GB-A-1 515 226**
**US-A-3 531 565**
**US-A-3 790 665**
**US-A-3 919 411**

**CHEMICAL ABSTRACTS, vol. 77, no. 15, 09-10-1972 abstract no. 96886g, page 23 COLUMBUS, OHIO (US)**
**DIE PHARMAZIE, vol. 28, no. 6, June 1973 BERLIN (DE) L. DRAGANOVA et al.: "Über Anwendungsmöglichkeiten des Knochenfettes als Komponente von Salbengrundlagen und Salben" pages 388-391**

(73) Proprietor: **CENTRAAL DIERGENEESKUNDIG INSTITUUT**
**Edelhertweg 15 P.O. Box 65**
**NL-8200 AB Lelystad (NL)**

(72) Inventor: **Bokhout, Bernard Adri**
**Weegbree 23**
**NL-2923 GJ Krimpen ad IJssel (NL)**
Inventor: **van der Heijden, Philippus Jan**
**Torenlaan 49**
**NL-3851 NP Ermelo (NL)**
Inventor: **Nabuurs, Marius Jozef Antonius**
**'t Jannendorp 13**
**NL-3791 VJ Achterveld (NL)**

(74) Representative: **van der Beek, George Frans et al**
**Nederlandsch Octrooibureau Johan de Wittlaan 15 P.O. Box 29720**
**NL-2502 LS 's-Gravenhage (NL)**

(56) References cited:
**DIE PHARMAZIE, vol. 29, no. 1, January 1974 BERLIN (DE) V. BOJANOVA et al.:**
**"Stabilisierung von pharmazeutischen Suspensionen" pages 43-45**
**Handbook of Experimental Immunology V. 3 Appendix A 3.1**

Courier Press, Leamington Spa, England.

## Description

Water-in-oil emulsions have been known as adjuvants for antigens. A well-known water-in-oil adjuvant is Freund's incomplete adjuvant which consists of an emulsion of physiological saline in a light mineral oil using Arlacel A® (impure form of the ester of mannitol and oleic acid) as the emulsifier Freund's complete adjuvant contains killed mycobacteria in the aqueous phase. Freund's complete and incomplete adjuvants are used as vehicles for antigens and provoke a substantial increase of the immune response in comparison with the effect of the antigen when administered in physiological saline. Complete Freund's adjuvant induces a general improvement of the immunity, among others by stimulating antibody production. Water-in-oil adjuvants such as Freund's incomplete adjuvant, have been used exclusively as vehicles for antigens, and have been administered by intradermal or intramuscular injection mostly.

It was found that antigen-free water-in-oil emulsions comprising an aqueous phase dispersed in a mineral oil by means of an emulsifier can be used successfully for the immuno-potentiation of animals if these emulsions are administered by intraperitoneal injection. Consequently, the invention relates to a water-in-oil emulsion for use in the potentiation of the immune system of animals by intraperitoneal injection, said emulsion being free of antigen and comprising an aqueous phase dispersed in a mineral oil by means of an emulsifier.

Surprisingly, intraperitoneal injection of the present antigen-free water-in-oil emulsions very rapidly potentiate the immunological response improving the mucosal immunity of the host animal.

In the water-in-oil emulsions according to the invention the aqueous phase may be simply water, or physiological saline. If desired, preserving agents suitable for parenteral administration, such as formalin, may be added. A preferred water phase is, for example, an aqueous solution containing 0.85% by weight of sodium chloride and 0.2% by volume of formalin.

Further, it was found that novel water-in-oil emulsions containing as an emulsifier a mixture of at least one sorbitan fatty acid ester and at least one polyoxyethylene sorbitan fatty acid ester are particularly effective for potentiation of the immune system of animals by intraperitoneal injection. Therefore, the invention relates to a novel injectable pharmaceutical composition comprising a water-in-oil emulsion of an aqueous phase dispersed in a mineral oil by means of an emulsifier consisting of a mixture of at least one sorbitan fatty acid ester and at least one polyoxyethylene sorbitan fatty acid ester, said composition being free of antigen.

Preferably, the emulsifier which, according to the invention, is a mixture of at least one sorbitan fatty acid ester and at least one polyoxyethylene sorbitan fatty acid ester, has HLB-value (hydrophile-lipophile balance) of 5.5—6.3.

Excellent results have been obtained with emulsifiers whose fatty acid radicals are derived from oleic acid, especially with mixtures of sorbitan trioleate and polyoxyethylene-20-sorbitan trioleate.

In the latter mixtures the volume ratio of sorbitan trioleate to polyoxyethylene-20-sorbitan trioleate is preferably 1:1 to 1.3:1 in view of the stability of the emulsion. The emulsifier may consist of other combinations of sorbitan fatty acid esters and polyoxyethylene sorbitan fatty acid esters as well. In that case the volume ratios are preferably chosen in such a way that the emulsifier has an HLB-value of 5.5—6.3.

Preferably, the volume ratio of emulsifier to mineral oil to aqueous phase is about 100:900:700—1000. Such emulsions show excellent stability and injectibility. Their viscosity is about 0.07—0.09 Pa.s (70—90 cP).

The mineral oil may be any mineral oil suitable for parenteral purposes. Good results have been obtained with Marcol 52® (Esso), a mixture of paraffins and cycloparaffins in the ratio of 63:37 having a chain length between 13 and 22 carbon atoms.

Preferred emulsifiers are well defined products having the best possible purity. In practice, good results have been obtained with a mixture of Span 85® (ICI, United States of America) and Tween 85® (ICI, United States of America).

The aqueous phase may be merely water, but preferably a salt such as sodium chloride is added in order to bring the osmotic pressure in agreement with that of the blood. Thus, the aqueous phase may be, for example a physiological salt solution. Further, preserving agents suitable for parenteral administration may be added, such as formalin etc. A preferred water phase is, for example, a solution of 0.85% by weight of sodium chloride and 0.2% by volume of formalin in distilled water.

The emulsions according to the present invention may be prepared in a way usual for water-in-oil emulsions. Generally, the mineral oil is first mixed with the emulsifier and then the aqueous phase is added. The emulsion is then prepared by vigorous mixing of the phases by means of a suitable agitating device, such as a colloid mill.

The invention is of great importance for the rearing of mammals, such as pigs, on an industrial scale.

The present emulsions have a high stability and are non-pyrogenic.

Although the immunopotentiating activity of the antigen-free emulsions according to the invention is not fully understood it is believed that the present emulsions induce immunity against pathogenic organisms present in the host or infecting the host shortly after the administration of the emulsion.

The following examples illustrate the invention.

Example I
Preparation of the emulsion

In a glass vessel 9 parts by volume of mineral oil (Marcol 52®) were thoroughly mixed with 1 part by volume of a mixture of sorbitan trioleate (Span 85®) and polyoxyethylene-20-sorbitan trioleate (Tween 85®) in a volume ratio of 54:46. Then the generator (No. T30G) of an Ultraturrax T45® (Janke & Kunkel) was placed under the surface of the mixture and 8 parts by weight of a water phase consisting of a physiological salt solution (0.85% by weight of NaCl+0.2% by volume of formalin) was gradually added to the mixture with continuous stirring at 10.000 rpm. After addition of the total amount of water phase the emulsification was continued for 1 minute. During the emulsification the vessel was cooled externally with tap-water. The temperature at the end of the process was about 45°C. Then the emulsion so obtained was poured into a beaker and the emulsion was cooled to room-temperature with stirring. The emulsion was brough into vial and stored at +4°C.

Stability tests

1. After storage for more than 1 year at 4°C the emulsions did not show any alterations, and the electrical conductivity of the emulsions appeared to show the original value.

2. After storage for more than 3 months at 37°C the present emulsions sometimes showed a thin clear fluid layer on top of the emulsion which was always less than 5% of the total volume.

3. After ten times shifting the temperature of the emulsions from 4°C to 37°C and back to 4°C the same fluid layer as described under 2, was sometimes observed.

4. The conductivity of the emulsions measured at 20°C was always smaller than 0.6 $\mu$ Siemens.

5. After centrifugation of the emulsions for 1 hour at 15000 g a thin fluid layer as described under 2 was observed.

Example II
Treatment of piglets with the emulsion according to example I.

a) Every week (on fridays) during a period of 6—11 months an intraperitoneal injection of 1 ml of the emulsion prepared in accordance with example I was given to half of the piglets (of 5 different pig-breeding farms) born in the 4th week preceding the injection week. On wednesdays following the injection these piglets were weaned. The treated piglets were not separated from the untreated ones.

In the year preceding the tests the average mortality of the weaned piglets in the same farms was 12%. During the period of treatment this mortality was about 2%. A significant difference in mortality between the untreated and treated animals could not be observed. An explanation of this phenomenon could be that the treatment of half of the animals would lower the "infection pressure" resulting in less infection in the untreated animals.

b) The following test was carried out in order to obtain a better insight in the protective activity of the present emulsion. The test was carried out during a period of 21 weeks in a pig-breeding farm having a production of about 100 piglets per week. All piglets to be weaned on wednesdays were treated on the preceding fridays according to the scheme shown in the following table. At the time of treatment the piglets were 4 to $4\frac{1}{2}$ weeks old. The emulsion according to example I was administered intraperitoneally in an amount of 1 ml. The physiological saline was also given intraperitoneally in an amount of 1 ml. Also, the table shows the number of piglets weaned in a particular week. From the beginning to the end of the experiment the 2267 piglets did not receive antibiotics. The piglets left the farm after a period of five weeks after weaning.

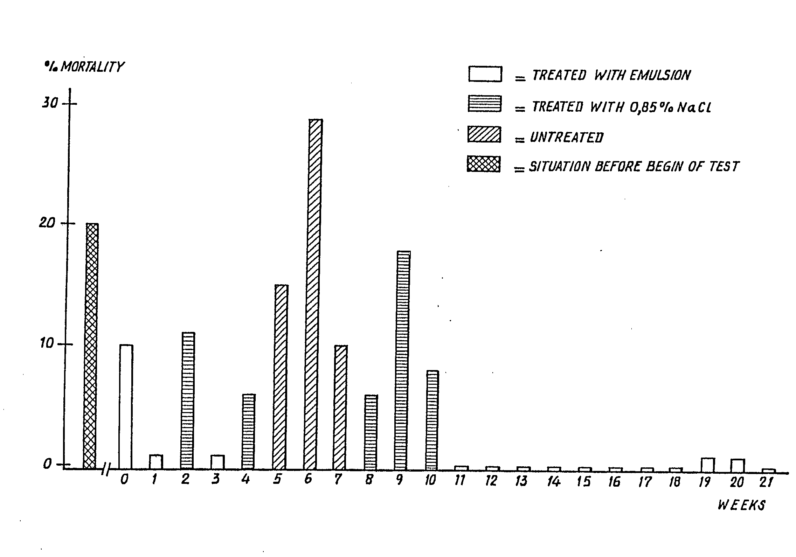

The emulsion treated, physiological saline treated and untreated animals were not separated from each other, but were added, every wednesday, to the existing population of about 500 weaned piglets. The percentage mortality in each of the groups during the five weeks after weaning was determined. This percentage is also mentioned in the table. The attached drawing shows the mortality in graphical form. The average mortality in the year preceding the test period was 20% (with use of antibiotics).

TABLE

| Week | Intraperitoneal injection with | Number of weaned piglets | Mortality in 5 weeks after weaning (%) |
|---|---|---|---|
| 0 | emulsion according to example I | 117 | 10 |
| 1 | emulsion according to example I | 83 | 1 |
| 2 | 0.85% NaCl | 79 | 11 |
| 3 | emulsion according to example I | 121 | 1 |
| 4 | 0.85% NaCl | 95 | 6 |
| 5 | nothing | 98 | 15 |
| 6 | nothing | 90 | 29 |
| 7 | nothing | 114 | 10 |
| 8 | 0.85% NaCl | 90 | 8 |
| 9 | 0.85% NaCl | 95 | 18 |
| 10 | 0.85% NaCl | 85 | 8 |
| 11—21 | emulsion according to example I | 1200 | 0—1 |

As shown by the table the mortality decreased strongly after the first injection with the emulsion, in spite of the fact that the animals did not receive antibiotics. After intraperitoneal injection of physiological saline in week 2 the mortality was 11%, whereas litters which had received the emulsion in week 1 or week 3 respectively showed a mortality of 1%. The percentage mortality of the animals treated in week 4 with physiological saline increased to 6% and the percentage mortality of the animals treated in weeks 5 to 7 inclusive increased to an average of about 18%. Injection with physiological saline of the animals weaned in weeks 8 to 10 inclusive resulted in an average mortality of 11%. None of the 324 piglets injected with the emulsion in weeks 11 to 13 inclusive died. This excellent result was maintained during the rest of the test. Only two piglets from litters injected with the emulsion in weeks 19 to 20 died from post-weaning disease.

The average mortality rates in piglets injected with the emulsion, with physiological saline and without treatment were 0.3%, 9.9% and 17.9% respectively.

No significant increase of body temperature was observed in any of the piglets after injection with the emulsion according to example I.

**Claims**

1. A water-in-oil emulsion for use in the potentiation of the immune system of animals by intraperitoneal injection, said emulsion being free of antigen and comprising an aqueous phase dispersed in a mineral oil by means of an emulsifier.

2. The emulsion of claim 1, in which the aqueous phase is water.

3. The emulsion of claim 1 in which the aqueous phase is physiological saline.

4. The emulsion of claim 1 in which the aqueous phase is 0.85% by weight of sodium chloride and 0.2% by volume of formalin in water.

5. Injectable pharmaceutical composition comprising a water-in-oil emulsion of an aqueous phase dispersed in a mineral oil by means of an emulsifier consisting of a mixture of at least one sorbitan fatty acid ester and at least one polyoxyethylene sorbitan fatty acid ester, said composition being free of antigen.

6. The composition of claim 5 in which the emulsifier has an HLB-value of 5.5—6.3.

7. The composition of claim 5 or 6, in which the fatty acid esters of the emulsifier are derived from oleic acid.

8. The composition of any of claims 5 to 7 in which the emulsifier consists of a mixture of sorbitan trioleate and polyoxyethylene-20-sorbitan trioleate.

9. The composition of claim 8, in which the volume ratio of sorbitan trioleate to polyoxyethylene-20-sorbitan trioleate is 1:1 to 1.3:1.

10. A composition of any of claims 5 to 9 in which the volume ratio of the emulsifier to mineral oil to aqueous phase is about 100:900:700—1000.

11. The composition of any of claims 5 to 10 in which the aqueous phase is 0.85% by weight of sodium chloride and 0.2% by volume of formalin in water.

12. The composition of any of claims 5 to 11 for use in the potentiation of the immune system of animals by intraperitoneal injection.

## Patentansprüche

1. Wasser-in-Öl-Emulsion für eine Verwendung zur Potenzierung des Immunsystems von Tieren durch interperitoneale Injektion, wobei die Emulsion frei von Antigen ist und eine wäßrige Phase aufweist, die in einem Mineral-öl mittels eines Emulgiermittels dispergiert ist.

2. Emulsion nach Anspruch 1, dadurch gekennzeichnet, daß die wäßrige Phase aus Wasser besteht.

3. Emulsion nach Anspruch 1, dadurch gekennzeichnet, daß die wäßrige Phase aus einer physiologischen Kochsalzlösung besteht.

4. Emulsion nach Anspruch 1, dadurch gekennzeichnet, daß die wäßrige Phase aus 0,85 Gew.-% Natriumchlorid und 0,2 Volumen-% Formalin in Wasser besteht.

5. Injizierbares pharmazeutisches Mittel aus einer Wasser-in-Öl-Emulsion einer wäßrigen Phase, die in einem Mineralöl mittels eines Emulgiermittels aus einer Mischung aus wenigstens einem Sorbitanfettsäureester und wenigstens einem Polyoxyethylensorbitanfettsäureester dispergiert ist, wobei das Mittel frei von Antigen ist.

6. Mittel nach Anspruch 5, dadurch gekennzeichnet, daß das Emulgiermittel einem HLB-Wert von 5,5 bis 6,3 besitzt.

7. Mittel nach Anspruch 5 oder 6, dadurch gekennzeichnet, daß die Fettsäureester des Emulgiermittels auf Ölsäure zurückgehen.

8. Mittel nach einem der Ansprüche 5 bis 7, dadurch gekennzeichnet, daß das Emulgiermittel aus einer Mischung aus Sorbitantrioleat und Polyoxyethylen-20-sorbitantrioleat besteht.

9. Mittel nach Anspruch 8, dadurch gekennzeichnet, daß das Volumeverhältnis von Sorbitantrioleat zu Polyoxyethylen-20-sorbitantrioleat 1:1 bis 1,3:1 beträgt.

10. Mittel nach einem der Ansprüche 5 bis 9, dadurch gekennzeichnet, daß das Volumenverhältnis des Emulgiermittels zu dem Mineralöl zu der wäßrigen Phase ungefähr 100:900:700:1000 beträgt.

11. Mittel nach einem der Ansprüche 5 bis 10, dadurch gekennzeichnet, daß die wäßrige Phase aus 0,85 Gew.-% Natriumchlorid und 0,2 Volumen-% Formalin in Wasser besteht.

12. Mittel nach einem der Ansprüche 5 bis 10 für eine Verwendung zur Potenzierung des Immunsystems von Tieren durch intraperitoneale Injektion.

## Revendications

1. Une émulsion eau dans l'huile pour utilisation dans le renforcement du système immunitaire d'animaux par injection intrapéritonéale, cette émulsion étant exempte d'antigène et comprenant une phase aqueuse dispersée dans une huile minérale au moyen d'un émulsifiant.

2. L'émulsion selon la revendication 1, caractérisée en ce que laphase aqueuse est de l'eau.

3. L'émulsion selon la revendication 1, caractérisée en ce que la phase aqueuse est du sérum physiologique.

4. L'émulsion selon la revendication 1, caractérisée en ce que la phase aqueuse est constituée de 0,85% en poids de chlorure de sodium et 0,2% en volume de formaline dans de l'eau.

5. Composition pharmaceutique injectable comprenant une émulsion huile dans eau d'une phase aqueuse dispersée dans une huile minérale au moyen d'un émulsifiant constitué par un mélange d'au moins un ester d'acide gras de sorbitan et au moins un ester d'acide gras de polyoxyéthylène sorbitan, cette composition étant exempte d'antigène.

6. La composition selon la revendication 5, caractérisée en ce que l'émulsifiant a un HLB de 5,5 à 6,3.

7. La composition selon la revendication 5 ou 6, caractérisée en ce que les esters d'acides gras de l'émulsifiant sont dérivés de l'acid oléique.

8. La composition selon l'une quelconque des revendication 5 à 7, caractérisée en ce que l'émulsifiant est constitué par un mélange de trioléate de sorbitan et de trioléate de polyoxyéthylène 20-sorbitan.

9. La composition selon la revendication 8, caractérisée en ce que le rapport en volume trioléate de sorbitan sur trioléate de polyoxyéthylene 20-sorbitan est de 1/1 à 1,3/1.

10. La composition selon l'une quelconque des revendications 5 à 9, caractérisée en ce que le rapport en volume émulsifiant sur huile minérale en phase aqueuse est d'environ 100/900/700 à 1000.

11. La composition selon l'une quelconque des revendications 5 à 10, caractérisée en ce que la phase aqueuse est constituée de 0,85% en poids de chlorure de sodium et 0,2% en volume de formaline dans de l'eau.

12. La composition selon l'une quelconque des revendications 5 à 11, pour utilisation dans le renforcement du système immunitaire d'animaux par injection intrapéritonéale.

0 059 521

% MORTALITY

□ = TREATED WITH EMULSION
▥ = TREATED WITH 0,85% NaCl
▨ = UNTREATED
▦ = SITUATION BEFORE BEGIN OF TEST

30
20
10
0

0 1 2 3 4 5 6 7 8 9 10 11 12 13 14 15 16 17 18 19 20 21

WEEKS

1